# FASCICULE DE BREVET EUROPEEN

(11) **EP 3 434 329 B1**
(45) Date de publication et mention de la délivrance du brevet: **14.07.2021**
(21) Numéro de dépôt: 18185026.4
(22) Date de dépôt: 23.07.2018
(51) Int. Cl.: A61Q 5/00, A61Q 7/00, A61K 8/9794, A61P 17/14

(54) **UTILISATION COSMÉTIQUE D'UN EXTRAIT DE TULIPE SUR LES CHEVEUX**
KOSMETISCHE ANWENDUNG EINES TULPENEXTRAKTS AUF DEM HAAR
COSMETIC USE OF A TULIP EXTRACT ON HAIR

(30) Priorité: 26.07.2017 FR 1757098
(43) Date de publication de la demande: 30.01.2019
(73) Titulaire: LABORATOIRE NATIVE, 75008 Paris (FR)
(72) Inventeur: BERNARD, Philippe, 45240 LA FERTE SAINT AUBIN (FR); CHOULOT, Jean-Christophe, 78120 RAMBOUILLET (FR); HAJEM, Neïla, 78290 CROISSY SUR SEINE (FR); HERLIN, Amélie, 77410 VILLEROY (FR); HIMBERT, Franck, 45000 ORLEANS (FR)
(74) Mandataire: Casalonga

(56) Documents cités:
- EP-A1- 1 915 997
- WO-A1-2015/006895
- CN-A- 101 028 234
- CN-A- 104 585 770
- FR-A1- 3 032 881
- JP-A- 2001 122 730
- US-A1- 2009 118 203
- DATABASE WPI Week 201552 2015 Thomson Scientific, London, GB; AN 2015-416604 XP002779728, & CN 104 585 770 A (LU X) 6 mai 2015 (2015-05-06)
- DATABASE BIOSIS [Online] BIOSCIENCES INFORMATION SERVICE, PHILADELPHIA, PA, US; 1988, NIEUWHOF M ET AL: "INHERITANCE OF FLOWER PIGMENTS IN TULIP TULIPA L", XP002779729, Database accession no. PREV198886069639 & NIEUWHOF M ET AL: "INHERITANCE OF FLOWER PIGMENTS IN TULIP TULIPA L", EUPHYTICA, vol. 38, no. 1, 1988, pages 49-54, ISSN: 0014-2336
- BUDZIANOWSKI ET AL: "Six flavonol glucuronides from Tulipa gesneriana", PHYTOCHEMISTRY, PERGAMON PRESS, GB, vol. 30, no. 5, 1 January 1991 (1991-01-01), pages 1679-1682, XP026631938, ISSN: 0031-9422, DOI: 10.1016/0031-9422(91)84233-I [retrieved on 1991-01-01]

## Description

La présente invention a pour objet l'utilisation cosmétique non thérapeutique d'un extrait issu de tulipe pour son action sur la croissance, la repousse et l'amélioration de la structure du cheveu, ainsi que sur la canitie chez un mammifère.

L'invention est aussi relative à l'utilisation d'une composition cosmétique non thérapeutique à base de cet extrait de tulipe.

Cette invention s'applique également au traitement cosmétique non thérapeutique de la chute des cheveux ainsi qu'à l'amélioration de la croissance, la repousse, la structure des cheveux et la canitie.

Le cheveu est constitué d'un follicule et d'une tige pilaire. Il est formé de compartiments individualisés, les uns d'origine dermique (gaine conjonctive, papille dermique), les autres d'origine épidermique (tige pilaire, glandes sébacées, gaines épithéliales). L'environnement matriciel et cellulaire de la papille du follicule pileux jour un rôle important dans la bonne santé du cheveu.

La pousse des cheveux s'effectue suivant un cycle comportant une phase de croissance (anagène), une phase de régression (catagène) et une phase de repos (télogène), aboutissant à la chute du cheveu, suivie d'un nouveau cycle. Le cycle normal de croissance des cheveux ou poils va de 2 à 6 ans. Chaque cheveu pousse d'environ 1 centimètre par mois au cours de cette phase. Environ 90 % des cheveux du cuir chevelu sont en phase de croissance (anagène). Environ 10% des cheveux du cuir chevelu, à un moment donné, sont dans une phase de repos (catagène). Après 2 à 3 mois, les cheveux qui étaient en repos chutent (télogène) pour être remplacés par la nouvelle repousse. Il est normal de perdre un certain nombre de cheveux (moins de 60 cheveux) chaque jour dans le cadre de ce cycle.

La perte des cheveux chez les êtres humains, chez la femme comme chez l'homme, est un processus normal. Toutefois, ce cycle peut être perturbé par diverses causes et la chute du cheveu peut ne pas être suivie d'une nouvelle pousse. L'alopécie peut aussi résulter de traitements médicamenteux, notamment de chimiothérapie de cancers, ou de radio- thérapies, ou encore de carences alimentaires. La perte des cheveux peut aussi être provoquée ou intensifiée par d'autres facteurs tels que les changements hormonaux (grossesse), le stress, un choc psychologique, une coloration agressive et excessive par certains produits capillaires, etc.

Quelques médicaments ont été proposés pour lutter contre la perte des cheveux, tels que le finastéride ou le minoxidil. Diverses compositions topiques destinées à stimuler la pousse des cheveux ou à en limiter la chute ont aussi été mises au point, par exemple des compositions à base de vitamine C, ou acide L-ascorbique, ou des dérivés.

Chez un humain, la couleur des poils et des cheveux est due à la mélanine. A différentes périodes de leur vie, notamment au cours du vieillissement, certaines personnes montrent une dépigmentation progressive de la chevelure, avec une diminution voire l'arrêt des processus de mélanogénèse dans les mélanocytes associés au bulbe pilaire. On parle de canitie. Au-delà du défaut de pigmentation, la canitie est également caractérisée par un cheveu dont le diamètre est plus gros, il pousse plus vite, il est moins brillant et plus difficile à discipliner. Enfin, c'est également un cheveu dont la fibre est perméable : il est plus résistant à la coloration, qui reste aujourd'hui le seul moyen de le masquer (The biology of hair diversity. G. E. Westgate et al. International Journal of Cosmetic Science, 35, pp329-336). Pour une prise en charge complète de la canitie, il convient donc de stimuler la pigmentation mais également de rétablir la structure du cheveu blanc.

L'exposition solaire et les rayonnements UV ont des effets délétères sur les cheveux, à la fois au niveau de la tige pilaire (oxydation et décoloration), mais aussi, et de façon plus dommageable, sur le bulbe du follicule, pouvant conduire à la chute du cheveu. La récupération ou la stimulation d'une pigmentation du follicule est susceptible de limiter la chute du cheveu ou de stimuler sa repousse, notamment dans des conditions d'exposition solaire intensive.

Par ailleurs, les cheveux font partie d'un système complexe appelé follicule pilo-sébacé. Il se compose de 95% de kératine et de KAP (Keratine Associated Proteins), qui sont associées aux kératines capillaires.

En effet, les cytokératines I et II se lient en spirales l'une à l'autre pour former des filaments intermédiaires (IF).

Les KAPs sont présents dans la matrice, autour des IF. On parle donc de protéines de matrice.

Le rôle des KAPs est ainsi de lier les IF entre eux. Ces protéines représentent le second composant majoritaire de la fibre après les kératines et elles jouent un rôle crucial pour la force de la tige capillaire.

De plus, au cours du vieillissement, on note un déclin de la synthèse de KAPs, ce qui explique les défauts de structure du cheveu chez les sujets âgés aboutissant à la formation d'une fibre cassante, fine...

Le follicule est constitué également de graisses (triglycérides, cire, cholestérol, phospholipides, scalène), de minéraux (fer, magnésium, zinc, cuivre, plomb), d'eau et de mélanines (eumélanine dans les cheveux bruns et phéomélanine dans les cheveux roux, blonds ou châtains dorés).

Dans les cheveux, on peut distinguer la partie visible externe du follicule pilifère appelée tige, la partie interne appelée racine, la partie profonde interne du follicule appelée bulbe où la matrice se trouve à la base. Les cellules de la matrice possèdent un noyau vésiculeux avec un cytoplasme dont l'indice mitotique est très élevé. Associées à d'autres protéines, les kératines se structurent en proto puis microfibrilles. Celles-ci sont enserrées dans une enveloppe externe, formée d'écailles de kératine et sont très rigides du fait de la présence de ponts disulfures entre les protéines de kératine. Les kératines et autres protéines associées aux kératines confèrent aux cheveux la résistance mécanique et l'élasticité relative des cheveux.

Il existe dans l'état de la technique de nombreuses compositions afin d'améliorer l'aspect et la résistance des cheveux, en particulier des compositions gainantes permettant d'améliorer l'aspect du cheveu. Toutefois, ces compositions ne permettent pas une amélioration de la structure et un renforcement de la fibre capillaire.

JP 2001/122730 décrit des compositions cosmétiques hydratantes contenant des extraits de plantes. Un certain nombre de plantes est cité dont *Tulipa gesneriana,* mais aucun extrait spécifique n'est cité.

CN 20131532095 décrit des capsules pour maintenir la couleur noire des cheveux ainsi que leur méthode de préparation. Ces capsules comprennent entre autres un extrait de tulipe sans préciser le type de tulipe utilisé.

WO 2015/006895 décrit une préparation médicinale afin de stimuler la repousse des cheveux. Ces formulations comprennent plusieurs plantes mais la tulipe n'est pas décrite. EP 1 915 997 décrit une composition pour la croissance des cheveux comprenant au moins un extrait semi mature de soja. Ces compositions peuvent également comprendre *Tulipa gesneriana* mais aucun extrait spécifique n'est cité.

CN 101028234 décrit une lotion antibactérienne pour animaux de compagnie contenant une *Tulipa gesneriana* et permettant de traiter les poils de ces animaux par leur action antimicrobienne anti-inflammatoires antibactérienne antiallergique et adoucissante de la peau. Cependant, aucun extrait de *Tulipa gesneriana* n'est décrit dans ce document.

US 2009/A0118203 décrit l'administration de compositions comprenant de la quercétine et du kaempférol agissant sur la tautoisomérase DOPAchrome pour protéger et/ou régénérer les mélanocytes des follicules pileux et pour limiter et ou arrêter le développement de canitie. FR 3032881 décrit une composition pour le traitement de l'alopécie, cette composition contenant au moins de la quercétine et de l'acide laurique. Cependant, aucun extrait de tulipe n'est cité dans ce document.

L'article de Nieuwhof et al., « Inheritance of flower pigments in tulip Tulipa L. », Euphytica, vol. 38, no. 1, 1988, pages 49-54, décrit les pigments présents dans la tulipe, et montre que la quercétine et le kaempférol sont présents dans cette plante.

L' article de Budzianowski et al, "Six flavonol glucuronides from Tulipa gesneriana", Phytochemistry, vol. 30, no. 5, 1991, pages 1679-1682, enseigne que les périanthes de Tulipa Geseneriana comprennent de la quercetine et du kaempferol.

Il existe donc un réel besoin de trouver des compositions efficaces pour lutter contre la chute de cheveux, améliorer la croissance, la repousse et la structure des fibres capillaires, ainsi qu'un traitement global pour une prise en charge complète de la canitie avec un effet anti-âge sur la structure de la fibre et une action sur la pigmentation, palliant l'ensemble des défauts, inconvénients et obstacles de l'art antérieur cités ci-dessus.

La demanderesse a mis au point l'utilisation cosmétique non thérapeutique d'extrait de tulipe pour les cheveux.

Un autre objet est constitué par les applications de cet extrait issu de tulipe dans le cadre d'un traitement cosmétique non thérapeutique visant à lutter contre la chute de cheveux, améliorer la repousse, la structure et la croissance du cheveu ainsi que la stimulation de la mélanogénèse et la pigmentation des cheveux ayant tendance au blanchissement.

D'autres objets, caractéristiques, aspects et avantages de l'invention apparaîtront encore plus clairement à la lecture de la description et des exemples qui suivent.

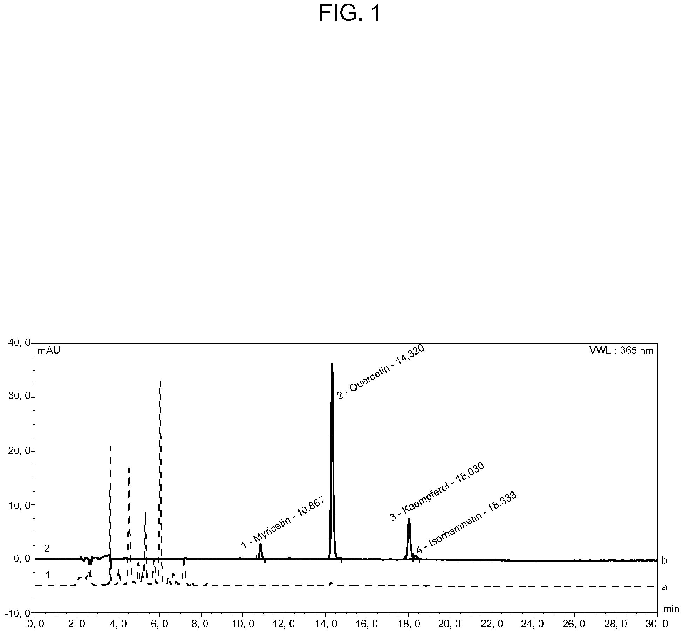

La figure 1 est un chromatogramme de l'extrait avant (a en pointillés) et après hydrolyse (b en traits pleins).

Dans la présente invention, par « extrait » de tulipe, on entend un extrait obtenu à partir de la plante et/ou d'une partie de la plante. II peut s'agir par exemple d'un extrait obtenu à partir de fleurs ou sommités fleuries, feuilles, graines, racines, tiges, graines, péricarpe de tulipe.

L'extrait utilisé dans l'invention est issu de tulipe de l'espèce *Tulipa gesneriana*. Il est riche en sucres et en composés phénoliques. Il est issu des pétales et comprend, en poids, de 20 à 70% de sucres et de 15 à 30% de composés phénoliques, dont au moins 8% de ces composés phénoliques sont des flavonoïdes, par rapport au poids total de l'extrait sec.

Les sucres sont principalement des monosaccharides et/ou des polysaccharides.

De préférence, cet extrait comprend, outre des monosaccharides et des polysaccharides, des flavonoïdes comprenant de 50 à 90% de quercétine et de 10 à 50% de kaempférol en poids par rapport au poids total de l'extrait sec. Plus préférentiellement, cet extrait comprend outre des monosaccharides et des polysaccharides, des flavonoïdes comprenant de 65 à 85% de quercétine et de 15 à 35% de kaempférol en poids par rapport au poids total de l'extrait sec.

Un extrait particulièrement préféré est défini par les chromatogrammes de la figure 1.

L'extrait utilisé dans la présente invention peut se présenter sous forme de poudre.

L'extrait issu de tulipe de la famille de *Tulipa gesneriana* selon l'invention est utile en cosmétique pour son action sur la croissance et la repousse des cheveux ainsi que pour l'amélioration de la structure des cheveux chez un mammifère.

L'extrait issu de tulipe de la famille de *Tulipa gesneriana* selon l'invention est également utile pour son action sur la canitie chez un mammifère.

Le procédé d'obtention de l'extrait selon l'invention est le suivant : l'extrait est obtenu à partir de tulipe et plus particulièrement de tulipe de l'espèce *Tulipa gesneriana,* et de préférence une tulipe sombre : de couleur pourpre.

Le procédé d'extraction permettant l'extraction sélective d'un extrait issu de tulipe de l'espèce *Tulipa gesneriana* selon l'invention est caractérisé par le fait que l'on procède à une ou plusieurs macérations, l'extrait liquide est clarifié, filtré, puis le précipité est ensuite séché pour obtenir l'extrait.

Seules les pétales de la tulipe sont utilisés pour la préparation de l'extrait.

De préférence, la ou les macérations s'effectuent dans un mélange eau/éthanol. Dans un mode de réalisation de ce procédé, le mélange pour la macération est composé de 70% d'eau stérile et de 30% d'éthanol. Les extraits sont obtenus à partir du procédé précédemment décrit à partir de tulipe de l'espèce *Tulipa gesneriana.*

Les compositions cosmétiques utilisées dans l'invention, comportent dans un milieu cosmétiquement acceptable, au moins un extrait selon l'invention, issu de tulipe de l'espèce T*ulipa gesneriana* autrement appelée tulipe des jardins, une espèce de plante de la famille des liliacées.

L'invention est également relative à l'utilisation cosmétique non thérapeutique de compositions cosmétiques comprenant au moins un tel extrait dans un milieu cosmétiquement acceptable et appropriées pour une utilisation topique chez un mammifère, préférablement un humain. Ces compositions sont utiles pour améliorer la repousse et la croissance du cheveu chez un mammifère, préférablement un humain.

Ces compositions sont également utiles pour améliorer la structure du cheveu chez un mammifère.

Les compositions utilisées dans l'invention agissent sur la canitie en stimulant la mélanogénèse et améliorant la pigmentation des cheveux ayant tendance au blanchissement chez un humain.

De telles compositions peuvent être utilisées à titre préventif ou curatif.

La composition utilisée dans l'invention est adaptée à une application topique sur les cheveux et comprend donc un milieu cosmétiquement acceptable, c'est-à-dire compatible avec les cheveux et la peau. Il s'agit d'un milieu qui présente une couleur, une odeur et un toucher agréables et qui ne génère pas de désagréments inacceptables (picotements, tiraillements, rougeurs), susceptibles de détourner le consommateur d'utiliser cette composition.

La composition utilisée dans l'invention peut comprendre avantageusement une phase aqueuse. La composition peut comprendre de l'eau en une teneur allant de 10% à 90% en poids par rapport au poids total de la composition, de préférence allant de 50% à 70% en poids par rapport au poids total de la composition.

La composition utilisée dans l'invention peut se présenter sous toutes les formes galéniques normalement utilisées pour une application topique sur les cheveux, notamment sous forme d'une émulsion huile-dans-eau ou eau-dans-huile ou multiple, éventuellement gélifiée, d'une émulsion siliconée, d'une microémulsion ou nanoémulsion, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol.

Avantageusement, la composition utilisée dans l'invention est sous forme d'une émulsion huile dans eau ou d'un gel, une crème ou un sérum.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les agents hydratants (tels que la glycérine, le propylène glycol, le butylène glycol, le pentylène glycol, l'hexylène glycol, le dipropylène glycol, le diéthylène glycol), les conservateurs, les antioxydants, les solvants, les parfums, les charges, les pigments, les filtres hydrophiles, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition selon l'invention est une émulsion, la proportion de la phase grasse peut aller de 5 à 50 % en poids, et de préférence de 5 à 30 % en poids par rapport au poids total de la composition.

Comme matières grasses utilisables dans l'invention, on peut utiliser les huiles minérales, les huiles d'origine animale, les huiles de synthèse, les huiles siliconées et les huiles fluorées. On peut aussi utiliser comme matières grasses des acides gras, des cires et des gommes et en particulier les gommes de silicone. De préférence, les compositions capillaires de l'invention sont sous forme de lotion, notamment pour pulvérisation, crème ou gel.

Les émulsionnants et les coémulsionnants éventuellement utilisés dans la composition sous forme d'émulsion sont choisis parmi ceux classiquement utilisés dans le domaine considéré. Ces émulsionnants et coémulsionnants sont de préférence présents, dans la composition, en une proportion allant de 0,3 à 20 % en poids, et de préférence de 0,5 à 5 % en poids par rapport au poids total de la composition. Comme émulsionnants et coémulsionnants utilisables dans l'invention, il est particulièrement avantageux d'utiliser les esters d'acide gras et de polyol tels que le stéarate de PEG-100, le stéarate de PEG-50 et le stéarate de PEG-40, le tristéarate de sorbitane, les stéarates de sorbitane oxyéthylénés disponibles sous les dénominations commerciales Tween 20 ou Tween 60, par exemple et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Bien entendu, l'homme de l'art veillera à choisir le ou les éventuels composés à ajouter aux compositions selon l'invention, ainsi que leur concentration, de manière telle que les propriétés avantageuses attachées intrinsèquement aux compositions conformes à l'invention ne soient pas, ou substantiellement pas, altérées par l'addition envisagée.

L'invention est également relative à un procédé de traitement cosmétique non thérapeutique des cheveux comprenant au moins une étape d'application sur les cheveux d'un mammifère de la composition selon l'invention pour son action sur la croissance et la repousse des cheveux ainsi que sur la structure du cheveu.

L'invention est également relative à un procédé de traitement cosmétique non thérapeutique des cheveux comprenant au moins une étape d'application sur les cheveux d'un mammifère de la composition selon l'invention pour lutter contre la chute de cheveux.

L'invention est également relative à un procédé de traitement cosmétique non thérapeutique des cheveux comprenant au moins une étape d'application sur les cheveux d'un mammifère de la composition selon l'invention pour lutter contre la canitie.

L'application sur les cheveux peut être réalisée en fonction de la forme galénique utilisée. II peut s'agir, par exemple d'une simple application sur les cheveux ou d'une application accompagnée, par exemple, d'un massage avec la composition de la présente invention. L'application est de préférence réalisée avec une quantité suffisante de la composition afin que toute la surface des cheveux à traiter soit traitée. II peut s'agir par exemple d'une application classique, comme une crème sur les cheveux. II peut s'agir par exemple aussi d'une application pour former un masque traitant. L'application peut être toute application souhaitée par l'utilisateur/l'utilisatrice, par exemple une application quotidienne, biquotidienne, hebdomadaire et/ou bimensuelle. Il peut s'agir par exemple d'une application une fois par jour, deux fois par jour ou plus.

L'invention sera maintenant illustrée à l'aide des exemples suivants.

### Exemple 1 : Obtention de l'extrait brut de tulipe selon l'invention

Un kilogramme de pétales de tulipe (*Tulipa gesneriana*) est mis à macérer à température ambiante pendant 24h sous agitation mécanique dans 10 litres d'une solution composés de 30% d'éthanol (Sigma, France) dans de l'eau stérile. La solution est ensuite clarifiée à l'aide d'un filtre à cadres creux (Colombo, Rover Pompe, Italie) avec un seuil de coupure de 10 à 15 µm. le filtrat est ensuite refiltré sur un filtre qui possède un seuil de coupure de 0.15 à 0.25 µm. l'éthanol est ensuite éliminé par filtration sous vide (Bûchi R-153, Suisse). On obtient une solution de 80 grammes d'extrait brut de tulipe.

### Exemple 2 : Caractérisation de l'extrait brut

L'extrait brut de tulipe de l'exemple 1 obtenu à partir de tulipe (*Tulipa gesneriana*) de couleur pourpre foncé a été analysé et les sucres totaux de cet extrait ont été dosés par la méthode au résorcinol.

L'extrait brut de tulipe selon l'invention présente une teneur en sucres totaux de 66,3% en équivalent glucose.

L'extrait est ensuite analysé en HPLC-UV avant et après hydrolyse acide. Avant l'étape d'hydrolyse acide, on observe les flavonoïdes aglycones présents. Après l'étape d'hydrolyse, on met en évidence les génines correspondant aux flavonoïdes glycosilés. Les chromatogrammes de l'extrait avant et après hydrolyse sont présentés dans la Figure 1 :
L'extrait de tulipe analysé avant hydrolyse est constitué principalement de composés sous forme glycosylée élués en début de gradient (Hariri E. B., Salle G., Andary C , Protoplasma, 1991, 162 (1) pp 20-26).

L'extrait brut de tulipe contient sous formes libre et glycosylée les noyaux flavonoïdiques suivants : la quercétine, le kaempférol, la myricétine et l'isorhamnétine.

La quercétine représente 70% de la totalité des noyaux flavonoidiques présents dans l'extrait testé. Le kaempférol est le deuxième noyau le plus présent (environ 20%) tandis que la myricétine et l'isorhamnétine représentent chacune environ 5% des composés formés sur un noyau flavone/flavonol.

Des composés non flavonoïdiques sont observés après hydrolyse. Un premier composé possède un spectre UV caractéristique d'une anthocyane (maxima à 276 et 525 nm). Un second composé (temps de rétention = 8,47 minutes) dont l'unique maximum d'absorbance est 290nm est un tanin. Ces pics de composés n'absorbant pas à 350nm n'apparaissent pas sur la Figure 1.

L'extrait est analysé en chromatographie liquide en phase inverse et détecté par une barrette de diode DAD-UV ainsi que par un spectromètre de masse haute résolution (HRMS).

Une colonne WATERS Acquity BEH C18 -150 x 2,1mm - 1,7µm est utilisée avec un débit de 0,2 mL/min à la température de 30°C. La concentration injectée est de 1000 ppm dans du méthanol comme solvant d'injection.

Le gradient d'élution est le suivant :

| Temps (min) | % acide formique 0,1% | % (methanol + acide formique 0,1%) |
|---|---|---|
| 0 | 90 | 10 |
| 3 | 90 | 10 |
| 30 | 20 | 80 |
| 35 | 20 | 80 |

L'extrait possède un riche profil UV à 254nm.

Pour le composé majoritaire observé en UV, les données obtenues en HRMS indiquent que la génine a une masse de 302,04122 g/mol, ce qui correspond à la masse monoisopique d'une pentahydroxyflavone dans le groupe des aglycones de flavonoïdes. L'analyse de l'extrait après hydrolyse détermine que c'est la quercétine. Les données de fragmentation obtenues en AutoMS/MS permettent de déterminer que les monosaccharides portés sont un hexose et un désoxyhexose.

La molécule sous forme sodée est fragmentée en AutoMS/MS. On observe un premier ion à 487,07348 g/mol, qui est la molécule ayant perdu le désoxyhexose. C'est donc l'hexose qui est lié à la génine. Un second ion fragment à 331.099217590 g/mol est observé. Cet ion représente la perte de la génine. Ceci indique que les deux unités glycosilées sont liées entre elles et que le composé est une quercétine diglycosilée sur une seule position avec l'hexose sur la génine et le désoxyhexose en position terminale.

Ainsi, les composés identifiés dans l'extrait brut de tulipe selon l'exemple 1 sont en grande partie des flavonols glycosilés, dont la rutine 7-o-beta-d-glucopyranoside, la populnin-3-O-rutinoside, l'isoquercitrine (ou quercétine 3-O-glucoside), le kaempférol-3-O-rutinoside, le kaempférol 3-O et/ou 7-O-glucoside, et la 3-o-(2(g)-alpha-1-rhamnopyranosylrutinoside quercimeritrine.

### Exemple 3 : Extrait prêt à l'emploi

720 grammes de maltodextrines (Tate&Lyle, Slovaquie) sont dissoutes dans l'extrait brut de l'exemple 2, puis la solution obtenue est atomisée (Bûchi 190, Suisse) et l'extrait sec final est obtenu.

Environ 650 grammes d'extrait de tulipe selon l'invention sont ainsi obtenus sous la forme d'une poudre fine de couleur violette.

### Exemple 4 : Evaluation de l'activité de l'extrait selon l'invention sur des explants de cuir chevelu ex vivo.

12 explants rectangulaires de 0.6x1.7 cm ont été préparés à partir d'une plastie de cuir chevelu d'un homme âgé de 63 ans de type caucasien.

Les explants ont été mis en survie en milieu BEM (BIO-EC's Explants Medium - BIO-EC Longjumeau à 37°C en atmosphère humide, enrichie de 5 % de CO2.

| Lots | Traitements | Nombre d'explants | Arrêts |
|---|---|---|---|
| T0 | - | 3 | J0 |
| T | - | 3 | J7 |
| P1 | Extrait de l'exemple 3 à 0.1% | 3 | J7 |
| P2 | Extrait de l'exemple 3 à 0.01% | 3 | J7 |

L'extrait selon l'exemple 3 de l'invention a été dilué dans de l'eau distillée stérile à 0.1% (lot P1) ou 0.01% (lot P2) et filtré au moyen d'un filtre possédant des pores de 0.22 µm, avant utilisation. L'extrait a ensuite été appliqué à J0, J2, et J4 en application topique à la dose de 2µl par explant (2mg/cm²) et étalé à l'aide d'une petite spatule.

Les explants du lot T n'ont reçu aucun traitement excepté le renouvellement du milieu de culture pour moitié (0.75mL) à J2 et J4.

A J0, 3 explants du lot T0 ont été prélevés et coupés en deux. Une partie a été fixée dans une solution de formol tamponnée et la 2ème partie a été congelée à -80°C.

A J7, 3 explants de chaque lot ont été prélevés et fixés de la même manière.

Après 24h dans le formol tamponné, les prélèvements ont été déshydratés et imprégnés en paraffine à l'aide d'un automate de déshydratation Leica TP 102. Ils ont été mis en bloc à l'aide d'une station d'enrobage Leica EG 1160.

Des coupes de 5 µm d'épaisseur ont été réalisées à l'aide d'un microtome type Minot, Leica RM2125. Des coupes congelées de 7 µm ont également été réalisées à l'aide d'un cryostat Leica CM 3050. Toutes les coupes ont été montées sur des lames de verre histologiques Superfrost®.

Les observations microscopiques ont été réalisées en microscopie optique, à l'aide d'un microscope Leica type DMLB ou Olympus BX43. Les prises de vue ont été réalisées avec une caméra Olympus DP72 et le logiciel CellD.

Les observations ont été réalisées sur les différentes zones du follicule pileux décrites ci-dessous :
L'infundibulum pilaire (ou folliculaire) est la partie superficielle du follicule pileux comprise entre l'orifice folliculaire, qu'il inclut, et l'abouchement de la glande sébacée.

La gaine épithéliale externe du cheveu correspond aux couches de cellules jointives à la lame basale, donc les couches les plus proches du derme papillaire. La gaine épithéliale interne du cheveu correspond aux couches de cellules les plus proches de la tige pilaire.

Le bulge est formé par une sous-population cellulaire de la gaine épithéliale externe, localisée au niveau de la portion moyenne du follicule pileux. Au niveau du bulge, les kératinocytes sont relativement indifférenciés, biochimiquement et ultrastructurellement. Ce bulge interagit durant la phase télogène tardive, avec la papille dermique pour initier un nouveau follicule en anagène. Ces cellules du bulge possèdent un potentiel prolifératif important et sont responsables du maintien à long terme et de la régénération du tissu.

La lame basale correspond à la jonction entre les kératinocytes des couches externes et le derme papillaire adjacent.

La papille folliculaire est une structure dermique richement vascularisée se situant au niveau du bulbe. Les cellules du bulbe au contact de la papille dermique, constituent la zone matricielle. Ces cellules matricielles reçoivent de la papule les informations nécessaires à leur différentiation en cellules corticales, qui se chargent progressivement en kératine au fur et à mesure de leur ascension. Ces cellules contiennent également le pigment mélanique.

La morphologie générale a été examinée sur des coupes en paraffine colorées au trichrome de Masson variante de Goldner.

Les explants sont globalement identiques.

Les explants ont ensuite été immunomarqués par différents anticorps. Les marquages ont été évalués par un examen microscopique et un comptage des cellules positives dans l'épiderme et dans les bulbes, de la manière suivante :
- Pour chaque explant, les cellules positives sont comptées sur toute la longueur de l'épiderme et le nombre est rapporté par longueur d'épiderme (nb/cm).
- Pour chaque explant, les cellules positives sont comptées dans tous les bulbes qui sont observables, et le nombre est rapporté par surface de bulbe (nb/mm²).

### a)Immunomarquage de PDGFR-alpha

Les facteurs de croissance dérivés des plaquettes « Platelet-derived growth factors » (PDGFs) agissent via leurs récepteurs tyrosine kinase et sont des mitogènes majeurs de beaucoup de cellules d'origine mésenchymateuse, incluant les fibroblastes et les cellules musculaires lisses. Grâce à leur rôle dans l'amplification des réponses migratoires et prolifératives et dans la synthèse de la matrice extracellulaire (MEC) de ces cellules, ces facteurs sont des régulateurs clé dans les fonctions biologique et pathologique telles que le remodelage des tissus, la cicatrisation et la fibrose. *In vitro,* les isoformes de PDGFR sont connues pour être des activateurs efficaces de la prolifération, de la migration et de la survie des fibroblastes.

En 2011, dans le tissu adipeux de souris, on a identifié des cellules souches, les préadipocytes, qui sont émettrices de signaux moléculaires stimulant les cellules souches quiescentes du follicule pileux et entrainant la croissance du poil chez la souris. Sur l'homme, l'activation de ces cellules souches du tissu adipeux est corrélée à celle des cellules souches de la peau.

En effet, chez les hommes dont la calvitie est héréditaire, les cellules souches du follicule pileux sont présentes à un état dormant et nécessiteraient des signaux provenant du tissu cutané pour redevenir actives. L'activation ces préadipocytes est donc corrélée à celle des follicules pileux.

Enfin, une étude sur des souris transgéniques a permis de démontrer que le facteur de croissance PDGF, synthétisé par les préadipocytes, est l'une des molécules qui permet de stimuler la croissance capillaire.

Le PDGFR-alpha a été marqué sur coupes paraffine formol avec un anticorps polyclonal anti-PDGFR-alpha, (Thermoscientific, ref. PA5-32545), dilué au 1/100ème dans du PBS-BSA pendant 1h à température ambiante, avec un système ImmPRESS anti-lapin HRP (Vector, MP7401), et révélé en VIP (Vector LAbs, SK-4600).

Le marquage a été évalué par un examen microscopique.

Un marquage est observé sur le bulbe des explants traités avec l'extrait selon l'invention. Le marquage est assez net sur l'épiderme, l'infundibulum, les gaines supérieures et inférieures ainsi que sur le bulge.

A T=0 et T=7, un marquage assez net est présent sur toute la surface du follicule.

Dans les explants traités par l'extrait selon l'invention, le marquage est net sur les cellules de papilles de bulbes.

L'extrait selon l'invention augmente donc la synthèse de PDGFR-alpha, impliqué pour son activation sur la prolifération, dans les cellules bulbaires.

### b) Immunomarquage de KAP11.1

Dans le cortex du cheveu, les filaments intermédiaires de kératine sont enchevêtrés dans une matrice interfilamenteuse, incluant les protéines associées aux kératines du cheveu (dont KAP11.1), qui sont essentielles pour la formation d'une tige pilaire résistante et rigide. Les protéines de la matrice incluent des kératines riches en sulfures et glycine-tyrosine. KAP11.1 a une expression différentielle selon la phase du cycle pilaire et semble impliquée dans la régulation du diamètre de la fibre.

KAP11.1 a été marquée sur coupes congelées avec un anticorps polyclonal anti-KRTAP11-1, (SAN Signalway antibody, ref. 43919), dilué au 1/200ème dans du PBS-BSA pendant 1h à température ambiante, et révélée avec AlexaFluor AF488 (Lifetechnologies A11008). Les noyaux ont été contre-colorés à l'iodure de propidium.

Le marquage a été évalué par un examen microscopique.

A T=0 et T=7, un marquage modéré est présent sur toute la surface du follicule à l'exception du bulbe.

Avec l'extrait selon l'invention, aux deux concentrations testées, l'épiderme et les gaines inférieures, au niveau de la gaine interne, montrent un marquage assez net.

L'augmentation de la quantité de KAP11.1 observée est essentielle pour la formation d'une tige pilaire résistante et rigide.

### c) Immunomarquage de la catalase

La peau est continuellement exposée à une multitude de stress environnementaux, tels que les UV, pouvant causer la formation d'espèces oxygénées réactives qui participent à la création d'un stress oxydant lorsque leur action n'est pas efficacement contrebalancée par suffisamment d'antioxydants. La glutathion peroxydase, les superoxydes dismutases et la catalase sont au nombre des enzymes antioxydantes produites par l'organisme.

De plus, la canitie peut avoir plusieurs causes, et en particulier survenir à la suite d'un phénomène oxydatif accru au niveau des follicules pileux. Ce paramètre est en effet déterminant pour le vieillissement cellulaire. Or les systèmes antioxydants sont justement altérés avec l'âge, et le mélanocyte au niveau du follicule n'est pas épargné. Ainsi, chez des donneurs âgés de 50 à 60 ans, on observe au sein des mélanocytes, et plus globalement dans l'ensemble du bulbe, une baisse de l'expression de la catalase, ainsi qu'une baisse de son activité. Ceci suggère que l'environnement des mélanocytes est plus oxydé avec l'âge, et que le mélanocyte est sensible à ce changement de son environnement. La catalase joue ainsi un rôle crucial dans le vieillissement intrinsèque et le processus de photo-vieillissement.

En effet, une diminution progressive de l'expression de la catalase au cours du vieillissement se traduit par une accumulation de peroxyde d'hydrogène, entre autres, dans les follicules des cheveux. Les données bibliographiques indiquent donc clairement le rôle primordial du déficit en catalase au niveau du follicule pileux dans le phénomène de blanchissement des cheveux.

La catalase a été marquée sur coupes congelées avec un anticorps polyclonal anti-catalase, (Santa Cruz, ref. sc-34280), dilué au 1/100ème dans du PBS-BSA et incubé pendant 1 nuit à 4°C, et révélé avec AlexaFluor AF488 (Lifetechnologies A11078). Les noyaux ont été contre-colorés à l'iodure de propidium.

Le marquage a été évalué par un examen microscopique.

A T=0 et T=7, un marquage assez net est présent sur l'épiderme les gaines supérieures et le bulbe, le reste du follicule étant faiblement marqué.

Avec l'extrait selon l'invention, aux deux concentrations testées, le bulge et les gaines inférieures montrent un marquage modéré.

Ainsi, l'extrait selon l'invention stimule l'expression de la catalase, agissant sur la dégradation des espèces oxygénées réactives, principales responsables de la dépigmentation du follicule.

### d) Immunomarquage de MC1-R

Les mélanines sont des macromolécules produites par les mélanocytes, par addition ou condensation de monomères formés à partir de tyrosine(eumélanine) ou de tyrosine et cystéine (phéomélanine), par l'enzyme tyrosinase.

Les mélanines sont responsables en particulier de la coloration des cheveux.

Dans la peau et le follicule pileux, MC1-R est un récepteur comportant 7 domaines transmembranaires couplés à la protéine G. MC1R prédomine dans les mélanocytes et répond à l'hormone α-MSH (alpha melanocyte stimulating hormone). Cette protéine stimule la pigmentation de la peau, en activant la synthèse de mélanine dans les mélanocytes. Elle est synthétisée par les fibroblastes et les kératinocytes. Elle active les mélanocytes en se fixant sur le récepteur MC1R présente sur la membrane des mélanocytes et induit l'expression d'enzymes responsables de la biogénèse des mélanosomes et de la synthèse de l'eumélanine dans les follicules pileux.

Le récepteur MC1R est donc nécessaire à la fois pour la synthèse puis pour la transmission du pigment de mélanine du mélanocyte vers le kératinocyte.

La voie de signalisation α-MSH/MC1R est également impliquée dans la régulation de l'immunité et de l'inflammation ayant un rôle anti-mélanome (Ferreira dos Santos Videira., I., & Lima Moura, D., F. (2013, An Bras Dermatol., Vol 88, pp 76-83.

Le MC1-R a été marqué sur coupes congelées avec un anticorps polyclonal anti-MC1-R, (Santa Cruz, ref. sc-28990), dilué au 1/50ème dans du PBS-BSA et incubé pendant 1 nuit à température ambiante, avec un système amplificateur streptavidine/biotine (Vector, Vectastain PK-7200), et révélé en VIP (Vector LAbs, SK-4600).

Le marquage a été évalué par un examen microscopique.

A T=0 et T=7, un marquage assez net à net est présent sur toute la surface du follicule.

Avec l'extrait selon l'invention, aux deux concentrations testées, le bulge, les gaines inférieures et le bulbe présentent un marquage net à très net.

Ainsi, l'augmentation de la quantité de MC1R observée en présence de l'extrait selon l'invention va avoir un effet positif sur la mélanogenèse, car les mélanocytes verront une augmentation de leur prolifération et de leur dendricité. L'extrait selon l'invention permet également une régulation de l'inflammation par l'activation de MC1-R, d'où une amélioration de l'ancrage du follicule pileux.

Ainsi, au-delà de cette action sur la pigmentation, MC1-R est également connu pour son rôle régulateur de l'unité mélano-kératinocytaire ; ainsi c'est une régulation globale du follicule pileux qui est mise en place par cet actif, comme le démontre les résultats suivants.

### f) Visualisation de la mélanine

La mélanine est synthétisée dans un organite spécifique, le mélanosome, qui est présent dans les dendrites des mélanocytes. Ces mélanocytes sont localisés dans la couche basale de l'épiderme, dans les bulbes de cheveux et dans les parois des follicules. La mélanine synthétisée dans les mélanosomes est transportée via les dendrites et accumulée dans les kératinocytes et mélanocytes, afin de protéger l'ADN épidermique des rayons UV.

La mélanine a été visualisée après imprégnation à l'argent selon la méthode de Masson, variante de Fontana.

L'imprégnation a été évaluée par un examen microscopique.

Sans extrait, à T=0 et T=7, une coloration modérée est observée au niveau de l'épiderme. Une coloration plus nette est observée au niveau du bulbe

Avec l'extrait selon l'invention, et surtout à la concentration la plus forte, on note une augmentation de la mélanine dans le bulbe.

Cette augmentation a un effet positif sur la mélanogenèse, car les mélanocytes verront une augmentation de leur prolifération et de leur dendricité. Au-delà de cette action sur la pigmentation, MC1-R est également connu pour son rôle régulateur de l'unité mélano-kératinocytaire ; ainsi c'est une régulation globale du follicule pileux qui est mise en place par cet actif.

### Exemple 5 : Shampooing pour cheveux

| Phase | INCI UE | % Matière |
|---|---|---|
| A | Aqua | 2,50 |
| | Sodium Lauryl Glucose Carboxylate | |
| | Lauryl Glucoside | |
| | Sodium Chloride | |
| | Sorbic Acid | |
| A | Aqua | 15,00 |
| | Sodium Lauroyl Sarcosinate | |
| | Sodium Benzoate | |
| A | Aqua | 3,00 |
| | Disodium Cocoamphodiacetate | |
| | Sodium Chloride | |
| A | Aqua | 2,00 |
| | Cocamidopropyl Betaine | |
| | Sodium Chloride | |
| | Glycerin | |
| | Coconut Acid | |
| A | Aqua | |
| | Coco-Glucoside | |
| | Glyceryl Oleate | |
| | Citric Acid | |
| | Tocopherol | |
| | Hydrogenated Palm Glycerides Citrate | |
| | Lecithin | |
| | Ascorbyl Palmitate | 12,00 |
| A | Peg-200 Hydrogenated Glyceryl Palmate | |
| | Aqua | |
| | Peg-7 Glyceryl Cocoate | 5,50 |
| A | Peg-15 Cocopolyamine | |
| | Aqua | 0,50 |
| A | Cananga Odorata Flower Oil | 0,05 |
| B | Aqua | |
| | Sodium Lauryl Glucose Carboxylate | |
| | Lauryl Glucoside | |
| | Sodium Chloride | |
| | Sorbic Acid | 5,00 |
| B | Peg-150 Distearate | 0,35 |
| C | Inulin | 0,50 |
| | Aqua | |
| C | Aqua | QSP 100 |
| C | Potassium Sorbate | 0,40 |
| C | Sodium Benzoate | 0,20 |
| C | Extrait selon l'exemple 3 de l'invention | 0,50 |
| D | Tetrasodium Edta | 0,05 |
| | Aqua | |
| D | Citric Acid | 0,50 |

Dans un fondoir/bain-marie, on introduit les ingrédients de la phase B et on fait fondre à 70°C (+/-5°C).

Dans la cuve de fabrication, on introduit successivement les ingrédients de la phase A sous agitation mécanique rapide et on mélange jusqu'à homogénéisation.

On incorpore la phase B sous agitation mécanique rapide et on mélange jusqu'à homogénéisation.

Dans une cuve, on incorpore successivement les ingrédients de la phase C dans l'eau et on mélange sous agitation mécanique moyenne jusqu'à homogénéisation. On incorpore alors la phase C dans la cuve de fabrication sous agitation mécanique rapide et on mélange jusqu'à homogénéisation.

On incorpore successivement les ingrédients de la phase D sous agitation mécanique rapide et on mélange pendant 20 mn minimum jusqu'à homogénéisation en vérifiant l'homogénéité du mélange.

### Exemple 6 : Lotion capillaire

| Phase | INCI UE | % Matière |
|---|---|---|
| A | Citrus Limon Peel Oil | 5,40 |
| A | Salvia Officinalis Oil | 1,30 |
| A | Rosmarinus Officinalis Leaf Oil | 1,30 |
| A | Melaleuca Leucadendron Cajaputi Oil | 1,30 |
| A | Cupressus Sempervirens Oil | 1,80 |
| A | Cananga Odorata Flower Oil | 2,10 |
| A | Serenoa Serrulata Fruit Extract | 3,20 |
| A | Alcohol Denat. | 10,60 |
| B | Vitis Vinifera Seed Extract | 1,10 |
| B | Lentinus Edodes Extract | 5,10 |
| B | Aqua | Qsp 100 |
| C | Rosmarinus Officinalis Leaf Extract | 39,28 |
| D | Extrait selon l'exemple 3 de l'invention | 2,00 |

Dans une cuve, on introduit successivement les ingrédients de la phase A sous agitation moyenne jusqu'à homogénéisation.

Dans un récipient, dissoudre les ingrédients de la phase B à 80°C (+/-5°C). On ajoute ensuite successivement les ingrédients des Phases C et D à 35°C sous agitation moyenne. On incorpore alors lentement, sous agitation rapide, la phase A dans la cuve de fabrication. On homogénéise sous agitation rapide pendant 20 minutes minimum et on vidange sur nylon 100 microns en poche stérile.

### Exemple 7 : Spray capillaire

| Phase | INCI UE | % Matière |
|---|---|---|
| A | Citrus Aurantium Dulcis Peel Oil | 5,00 |
| A | Lavandula Angustifolia Oil | 1,30 |
| A | Thymus Vulgaris Flower/Leaf Oil | 1,30 |
| A | Salvia Officinalis Oil | 1,30 |
| A | Rosmarinus Officinalis Leaf Oil | 2,30 |
| A | Balanites Aegyptiaca Kernel Oil | 1,60 |
| A | Moringa Pterygosperma Seed Oil | 2,30 |
| A | Serenoa Serrulata Fruit Extract | 3,20 |
| A | Polyglyceryl-2 Dipolyhydroxystearate | |
| | Lauryl Glucoside | |
| | Glycerin | |
| | Aqua | 1,00 |
| B | Lentinus Edodes Extract | 5,00 |
| B | Tryptophan | |
| | Methionine | |
| | Cysteine | 0,20 |
| B | Inula Helenium Extract | 5,00 |
| B | Aqua | Qsp 100 |
| D | Extrait selon l'exemple 3 de l'invention | 1,00 |
| D | Alcohol Denat. | 10,00 |

Dans un récipient en inox introduire dans l'ordre indiqué les ingrédients de la phase A, sous agitation manuelle.

Dans un autre récipient en inox, on dissout successivement dans la quantité d'eau indiquée à 50°C, tous les ingrédients sous agitation moyenne. Vers 30°C, on ajoute successivement et sous agitation, dans l'ordre indiqué, les ingrédients de la phase D et on maintient l'agitation pendant 15 mn.

### Exemple 8 : masque capillaire après shampooing

| Phase | INCI UE | % Matière |
|---|---|---|
| A | Aqua | QSP 100 |
| A | Phenoxyethanol | |
| | Chlorphenesin | |
| | Glycerin | 1,20 |
| A | Hydroxyethylcellulose | 2,00 |
| B | Tetrasodium Edta | |
| | Aqua | 0,01 |
| C | Cetearyl Alcohol | |
| | Behentrimonium Chloride | 5,50 |
| C | Cetyl Alcohol | 1,00 |
| C | Argania Spinosa Kernel Oil | |
| | Hydrogenated Argania Spinosa Kernel Oil | |
| | Tocopherol | 5,00 |
| D | Aqua | |
| | Cetrimonium Chloride | |
| | Citric Acid | 2,00 |
| D | Aqua | |
| | Polyquaternium-53 | |
| | Phenoxyethanol | 0,10 |
| E | Glycerin | |
| | Aqua | |
| | Hydrolyzed Ceratonia Siliqua Seed Extract | |
| | Zea Mays Starch | |
| | Sodium Benzoate | |
| | Potassium Sorbate | |
| | Guar Hydroxypropyltrimonium Chloride | 2,00 |
| | Polyquaternium-7 | |
| E | Extrait selon l'exemple 3 de l'invention | 0,50 |
| E | Aqua | |
| | Glycerin | |
| | Oryza Sativa Seed Protein | |
| | Phytic Acid | |
| | Oryza Sativa Extract | |
| | Gluconolactone | |
| | Sodium Benzoate | 1,50 |
| E | Parfum | 0,50 |

Dans un fondoir/bain-marie, on introduit les ingrédients de la phase C et on fait fondre à 75-80°C.

Dans la cuve de fabrication, on introduit l'eau de la phase A à 75-80°C. On incorpore successivement les autres ingrédients de la phase A sous agitation turbine rapide, puis on mélange sous vide sous agitation turbine moyenne et planétaire moyenne pendant 45 min minimum jusqu'à homogénéisation tout en maintenant à 75-80°C.

On incorpore successivement les ingrédients de la phase B sous agitation turbine moyenne et planétaire moyenne jusqu'à homogénéisation.

Dans la cuve de fabrication, à 75-80°C, par aspiration sous vide, on incorpore la phase C sous agitation turbine rapide. On mélange sous vide sous agitation turbine rapide et planétaire rapide pendant 15 min minimum et jusqu'à homogénéisation.

On commence alors le refroidissement sous vide sous agitation turbine moyenne et planétaire moyenne.

A 40-45°C, on incorpore successivement les ingrédients de la phase D et on mélange sous vide sous agitation planétaire rapide pendant 15 min minimum et jusqu'à homogénéisation.

A 30-35°C, par le trou d'homme, on incorpore successivement les ingrédients de la phase E sous agitation planétaire rapide. On mélange sous vide sous agitation planétaire moyenne pendant 10 min minimum jusqu'à homogénéisation.

On vérifie l'homogénéité du mélange et l'absence de grains avant vidange en poche stérile.

## Revendications

1. Utilisation cosmétique non thérapeutique d'un extrait issu de pétales de tulipe de la famille de *Tulipa gesneriana* comprenant en poids, de 20 à 70% de sucres et de 15 à 30 % de composés phénoliques, dont au moins 8% de ces composés phénoliques sont des flavonoïdes, par rapport au poids total de l'extrait sec, pour son action sur la croissance et la repousse des cheveux chez un mammifère.

2. Utilisation cosmétique non thérapeutique d'un extrait issu de pétales de tulipe de la famille de *Tulipa gesneriana* comprenant en poids, de 20 à 70% de sucres et de 15 à 30 % de composés phénoliques, dont au moins 8% de ces composés phénoliques sont des flavonoïdes, par rapport au poids total de l'extrait sec, pour son action sur l'amélioration de la structure des cheveux chez un mammifère.

3. Utilisation cosmétique non thérapeutique d'un extrait issu de pétale de tulipe de la famille de *Tulipa gesneriana* comprenant en poids, de 20 à 70% de sucres et de 15 à 30 % de composés phénoliques, dont au moins 8% de ces composés phénoliques sont des flavonoïdes, par rapport au poids total de l'extrait sec, pour son action sur la canitie chez un mammifère.

4. Utilisation selon l'une quelconque des revendications 1 à 3, d'un extrait de tulipe **caractérisé par le fait que** les sucres comprennent des monosaccharides et des polysaccharides.

5. Utilisation selon l'une quelconque des revendications 1 à 4, d'un extrait de tulipe **caractérisé par le fait que** les flavonoïdes comprennent en poids de 50 à 90 % de quercétine et de 10 à 50% de kaempférol.

6. Utilisation selon l'une quelconque des revendications 1 à 5, d'un extrait de tulipe **caractérisé par le fait que** les flavonoïdes comprennent en poids de 65 à 85 % de quercétine et de 15 à 35% de kaempférol.

7. Utilisation selon l'une quelconque des revendications 1 à 6, d'un extrait de tulipe **caractérisé par le fait que** la tulipe utilisée est une tulipe pourpre.

8. Utilisation selon l'une quelconque des revendications 1 à 7, d'un extrait de tulipe **caractérisé par le fait qu'**il se présente sous forme de poudre.

9. Utilisation selon l'une quelconque des revendications 1 à 8, d'un extrait de tulipe défini par les chromatogrammes de la figure 1 qui représentent l'analyse dudit extrait avant et après hydrolyse acide par chromatographie liquide en phase inverse et sa détection par une barrette de diode DAD-UV ainsi que par un spectromètre de masse haute résolution (HRMS).

10. Utilisation non thérapeutique d'une composition cosmétique non thérapeutique comprenant dans un milieu cosmétiquement acceptable, au moins un extrait tel que défini dans l'une quelconque des revendications 1 à 9 pour son action sur la croissance et la repousse des cheveux chez un mammifère.

11. Utilisation non thérapeutique d'une composition cosmétique non thérapeutique comprenant dans un milieu cosmétiquement acceptable, au moins un extrait tel que défini dans l'une quelconque des revendications 1 à 9 pour son action sur l'amélioration de la structure des cheveux chez un mammifère.

12. Utilisation non thérapeutique d'une composition cosmétique non thérapeutique comprenant dans un milieu cosmétiquement acceptable, au moins un extrait tel que défini dans l'une quelconque des revendications 1 à 9 pour son action sur la canitie chez un mammifère.

13. Procédé de traitement cosmétique non thérapeutique des cheveux d'un mammifère destiné à améliorer la croissance, la repousse et la structure des cheveux, consistant à appliquer sur lesdits cheveux une composition contenant au moins un extrait de tulipe selon l'une quelconque des revendications 1 à 9.

14. Procédé de traitement cosmétique non thérapeutique des cheveux d'un mammifère destiné à lutter contre la canitie, consistant à appliquer sur lesdits cheveux une composition contenant au moins un extrait de tulipe selon l'une quelconque des revendications 1 à 9.

15. Procédé de traitement cosmétique non thérapeutique des cheveux d'un mammifère destiné à lutter contre la chute de cheveux, consistant à appliquer sur lesdits cheveux une composition contenant au moins un extrait de tulipe selon l'une quelconque des revendications 1 à 9.

## Patentansprüche

1. Nicht-therapeutische kosmetische Anwendung eines aus den Blütenblättern einer Tulpe aus der Familie *Tulipa gesneriana* gewonnenen Extrakts, der in Gewicht 20 bis 70 % Zucker und 15 bis 30 % Phenolverbindungen umfasst, wobei mindestens 8 % dieser Phenolverbindungen Flavonoide sind, in Bezug auf das Gesamtgewicht des Trockenextrakts, für dessen Wirkung auf das Wachstum und das Nachwachsen der Haare bei einem Säugetier.

2. Nicht-therapeutische kosmetische Anwendung eines aus den Blütenblättern einer Tulpe aus der Familie *Tulipa gesneriana* gewonnenen Extrakts, der in Gewicht 20 bis 70 % Zucker und 15 bis 30 % Phenolverbindungen umfasst, wobei mindestens 8 % dieser Phenolverbindungen Flavonoide sind, in Bezug auf das Gesamtgewicht des Trockenextrakts, für dessen Wirkung auf die Verbesserung der Struktur der Haare bei einem Säugetier.

3. Nicht-therapeutische kosmetische Anwendung eines aus den Blütenblättern einer Tulpe aus der Familie *Tulipa gesneriana* gewonnenen Extrakts, der in Gewicht 20 bis 70 % Zucker und 15 bis 30 % Phenolverbindungen umfasst, wobei mindestens 8 % dieser Phenolverbindungen Flavonoide sind, in Bezug auf das Gesamtgewicht des Trockenextrakts, für dessen Wirkung auf das Ergrauen bei einem Säugetier.

4. Anwendung nach einem der Ansprüche 1 bis 3 eines Tulpenextrakts, **dadurch gekennzeichnet, dass** die Zucker Monosaccharide und Polysaccharide umfassen.

5. Anwendung nach einem der Ansprüche 1 bis 4 eines Tulpenextrakts, **dadurch gekennzeichnet, dass** die Flavonoide in Gewicht 50 bis 90 % Quercetin und 10 bis 50 % Kaempferol umfassen.

6. Anwendung nach einem der Ansprüche 1 bis 5 eines Tulpenextrakts, **dadurch gekennzeichnet, dass** die Flavonoide in Gewicht 65 bis 85 % Quercetin und 15 bis 35 % Kaempferol umfassen.

7. Anwendung nach einem der Ansprüche 1 bis 6 eines Tulpenextrakts, **dadurch gekennzeichnet, dass** die verwendete Tulpe eine Purpurtulpe ist.

8. Anwendung nach einem der Ansprüche 1 bis 7 eines Tulpenextrakts, **dadurch gekennzeichnet, dass** er in Form von Pulver vorliegt.

9. Anwendung nach einem der Ansprüche 1 bis 8 eines Tulpenextrakts, der von den Chromatogrammen der Figur 1 definiert ist, die die Analyse des Extrakts vor oder nach der Säurehydrolyse durch Umkehrphasen-Flüssigkeitschromatographie und dessen Detektion mittels einer Diodenanordnung DAD-UV sowie einem hochauflösenden Massenspektrometer (HRMS) darstellen.

10. Nicht-therapeutische Anwendung einer nicht-therapeutischen kosmetischen Zusammensetzung, die in einem kosmetisch akzeptablen Medium mindestens einen Extrakt nach einem der Ansprüche 1 bis 9 umfasst, wegen seiner Wirkung auf das Wachstum und das Nachwachsen der Haare bei einem Säugetier.

11. Nicht-therapeutische Anwendung einer nicht-therapeutischen kosmetischen Zusammensetzung, die in einem kosmetisch akzeptablen Medium mindestens einen Extrakt nach einem der Ansprüche 1 bis 9 umfasst, wegen seiner Wirkung auf die Verbesserung der Struktur der Haare bei einem Säugetier.

12. Nicht-therapeutische Anwendung einer nicht-therapeutischen kosmetischen Zusammensetzung, die in einem kosmetisch akzeptablen Medium mindestens einen Extrakt nach einem der Ansprüche 1 bis 9 umfasst, wegen seiner Wirkung auf das Ergrauen bei einem Säugetier.

13. Verfahren zur nicht-therapeutischen kosmetischen Behandlung der Haare eines Säugetiers, das dazu bestimmt ist, das Wachstums, das Nachwachsen und die Struktur der Haare zu verbessern, das darin besteht, auf die Haare eine Zusammensetzung aufzutragen, die mindestens einen Tulpenextrakt nach einem der Ansprüche 1 bis 9 enthält.

14. Verfahren zur nicht-therapeutischen kosmetischen Behandlung der Haare eines Säugetiers, das dazu bestimmt ist, das Ergrauen zu bekämpfen, das darin besteht, auf die Haare eine Zusammensetzung aufzutragen, die mindestens einen Tulpenextrakt nach einem der Ansprüche 1 bis 9 enthält.

15. Verfahren zur nicht-therapeutischen kosmetischen Behandlung der Haare eines Säugetiers, das dazu bestimmt ist, den Haarausfall zu bekämpfen, das darin besteht, auf die Haare eine Zusammensetzung aufzutragen, die mindestens einen Tulpenextrakt nach einem der Ansprüche 1 bis 9 enthält.

## Claims

1. Non-therapeutic cosmetic use of an extract from tulip petals of the *Tulipa gesneriana* family comprising by weight, from 20 to 70% sugars and from 15 to 30% phenolic compounds, of which at least 8% of these phenolic compounds are flavonoids, in relation to the total weight of the dry extract, for its action on the growth and the regrowth of hair in a mammal.

2. Non-therapeutic cosmetic use of an extract from tulip petals of the *Tulipa gesneriana* family comprising by weight, from 20 to 70% sugars and from 15 to 30% phenolic compounds, of which at least 8% of these phenolic compounds are flavonoids, in relation to the total weight of the dry extract, for its action on the improvement of the structure of hair in a mammal.

3. Non-therapeutic cosmetic use of an extract from tulip petals of the *Tulipa gesneriana* family comprising by weight, from 20 to 70% sugars and from 15 to 30% phenolic compounds, of which at least 8% of these phenolic compounds are flavonoids, in relation to the total weight of the dry extract, for its action on canities in a mammal.

4. Use according to any one of claims 1 to 3, of a tulip extract **characterised by** the fact that the sugars comprise monosaccharides and polysaccharides.

5. Use according to any one of claims 1 to 4, of a tulip extract **characterised by** the fact that the flavonoids comprise by weight from 50 to 90% quercetin and from 10 to 50% kaempferol.

6. Use according to any one of claims 1 to 5, of a tulip extract **characterised by** the fact that the flavonoids comprise by weight from 65 to 85% quercetin and from 15 to 35% kaempferol.

7. Use according to any one of claims 1 to 6, of a tulip extract **characterised by** the fact that the tulip used is a purple tulip.

8. Use according to any one of claims 1 to 7, of a tulip extract **characterised by** the fact that it is in powder form.

9. Use according to any one of claims 1 to 8, of a tulip extract defined by the chromatograms of Figure 1 that show the analysis of said extract before and after acid hydrolysis by reverse phase liquid chromatography and its detection by a diode array DAD-UV as well as by a high-resolution mass spectrometry (HRMS).

10. Non-therapeutic use of a non-therapeutic cosmetic composition comprising in a cosmetically acceptable medium, at least one extract such as defined in any one of claims 1 to 9 for its action on the growth and the regrowth of hair in a mammal.

11. Non-therapeutic use of a non-therapeutic cosmetic composition comprising in a cosmetically acceptable medium, at least one extract such as defined in any one of claims 1 to 9 for its action on the improvement of the structure of hair in a mammal.

12. Non-therapeutic use of a non-therapeutic cosmetic composition comprising in a cosmetically acceptable medium, at least one extract such as defined in any one of claims 1 to 9 for its action on canities in a mammal.

13. Method for non-therapeutic cosmetic treatment of hair of a mammal intended to improve the growth, the regrowth and the structure of hair, consisting of applying on said hair a composition containing at least one tulip extract according to any one of claims 1 to 9.

14. Method for non-therapeutic cosmetic treatment of hair of a mammal intended to combat canities, consisting of applying on said hair a composition containing at least one tulip extract according to any one of claims 1 to 9.

15. Method for non-therapeutic cosmetic treatment of hair of a mammal intended to combat hair loss, consisting of applying on said hair a composition containing at least one tulip extract according to any one of claims 1 to 9.
